# EUROPEAN PATENT APPLICATION

(11) **EP 4 129 208 A1**
(43) Date of publication of application: **08.02.2023**
(21) Application number: 21774929.0
(22) Date of filing: 19.03.2021
(51) Int. Cl.: A61B 17/11

(54) **MEDICAL INSTRUMENT SUITABLE FOR LIGATURE OR SIMILAR**

(30) Priority: 26.03.2020 JP 2020056373
(71) Applicant: Jichi Medical University, Tokyo 102-0093 (JP); Teijin Medical Technologies Co., Ltd., Osaka 530-0005 (JP)
(72) Inventor: KANEDA, Yuji, Shimotsuke-shi, Tochigi 329-0498 (JP); KAWABE, Yasuhiro, Osaka-shi, Osaka 530-0005 (JP); MIKAMI, Katsuhiro, Osaka-shi, Osaka 530-0005 (JP); HIRATA, Masumi, Osaka-shi, Osaka 530-0005 (JP)
(74) Representative: Ipsilon
(86) International application number: PCT/JP2021/011479
(87) International publication number: WO 2021/193464

(57) **Abstract**

A medical instrument, comprising a flexible strap portion having a proximal end and a distal end; a head portion distally extending from and connected to the distal end of the strap portion; and a tail portion proximally extending from and connected to the proximal end of the strap portion; wherein the head portion and the strap portion have a locking feature that allows the strap portion to be tightened to the head portion at a desired position, a loop having a desired size composed of a distal end side part of the strap portion and the head portion can be formed when tightened by the locking feature, and a plurality of features for keeping connected to thread are along the longitudinal direction on at least the distal end side part of the strap portion that forms the loop.

## Description

### TECHNICAL FIELD

The present invention relates to a medical instrument. More particularly, the present invention relates to a medical instrument that can be used to prevent the occurrence of various complications in partial resection and the like of living tissue (e.g., organ, organum) such as liver, pancreas and blood vessels, and is less invasive and suitable for ligation, suture, fixation, etc.

### BACKGROUND ART

Ligation is performed, for example, to pull and fix a tissue severed due to trauma, etc., to discontinue a lumen by tying it around blood vessel, fallopian tube or the like, to bind and fix a tissue to close hernia gate, etc., or to tie up a tissue to be removed to stop the blood flow and leading to necrosis or sloughing off. Various medical instruments for performing ligation or the others have been proposed.

For example, Patent Document 1 discloses a ligation band composed of a synthetic resin molded product having flexibility as a whole, the synthetic resin molded product comprising a tourniquet body having a predetermined length, wherein a retaining mesh portions are continuously formed within the range that serves as a tightening allowance in the length direction of the tourniquet body, non-slip ribs are continuously formed at appropriate locations on the tourniquet body over the formation range of the retaining mesh portions, furthermore, at the base end of the tourniquet body, a short tunnel shape with an insertion port on one side and a feeding port on another side is formed, a buckle part is integrally formed at an appropriate place on the inside, and a buckle portion is formed with a locking hook capable of non-removably locking the retaining mesh portion of the tourniquet body rolled in from the tip.

Patent Document 2 discloses a medical device for tissue ligation, comprising an elongated flexible band having a front side portion, a rear side portion, a tip portion, and a trailing end portion, wherein the band has a perforation portion and a cross rail portion defined within the band, a locking case connected to the trailing end portion of the band, wherein the locking case has a channel sized for receiving the band, and a locking member connected to the locking case, wherein the locking member is arranged in relation to the channel and is configured to connect with the perforation portion and the cross rail portion defined in the band, characterized in that the channel in the locking case includes an arching portion located on the opposite side of the locking member, the band becomes arched above the locking member when the locking member engages with the cross rail portion of the band, and it is configured to project at least partially into the arching portion.

Patent Document 3 discloses an organ stump treatment tool, for tying up the organ with a flat loop to ligate canal or cavity opened in the organ stump, comprising an elongate flexible first band portion having a distal end and a proximal end composed of a biodegradable and bioabsorbable polymer; an elongated flexible second band portion having a distal end and a proximal end composed of a biodegradable and bioabsorbable polymer; a first locking portion having a first ratchet pawl composed of a biodegradable and bioabsorbable polymer; wherein the first locking portion is formed at the distal end of the second band portion, and the distal end of the first band portion and the proximal end of the second band portion are joined; and at least one ratchet tooth that can be engaged with the first ratchet pawl is formed on the outer surface of the first band portion, and the flat loop is formed by engaging the ratchet tooth and the first ratchet pawl.

Patent Document 4 discloses a bone tying band composed of a biodegradable and bioabsorbable material for binding adjacent bone fragments by forming a tying loop across holes formed in each of the adjacent bone fragments, characterized in that the bone tying band comprises a strip portion having a length capable of forming the tying loop, and a hooking portion formed at one end of the strip portion so as to be accommodated in the hole of the bone fragment when the tying loop is formed and hooking the other end side of the strip portion allows the tying loop to be formed; a plurality of hooking grooves are formed on at least one side of the strip portion, the hooking portion is formed with a retaining hole into which the other end side of the strip portion is inserted, the retaining hole is provided with a hooking tooth that engages with the hooking groove of the strip portion to retain the strip portion.

### CITATION LIST

### PATENT LITERATURES

PATENT DOCUMENT 1 : JP 2004-298501 A
PATENT DOCUMENT 2 : JP 2015-523144 A
PATENT DOCUMENT 3 : WO 2019/039586 A1
PATENT DOCUMENT 4 : JP 2000-201941 A

### SUMMARY OF THE INVENTION

### PROBLEM TO BE RESOLVED BY THE INVENTION

An object of the present invention is to provide a medical instrument that can be used to prevent occurrence of various complications in partial resection and the like of living tissue (e.g., organ, organum) such as liver, pancreas and blood vessels, and is less invasive and suitable for ligation, suture, fixation, etc.

### MEANS FOR RESOLVING THE PROBLEM

As a result of intensive studies in order to solve the above problems, the present invention including the following embodiments has been completed.
[1] A medical instrument, comprising a flexible strap portion having a proximal end and a distal end; a head portion distally extending from and connected to the distal end of the strap portion; and a tail portion proximally extending from and connected to the proximal end of the strap portion; wherein the head portion and the strap portion have a locking feature that allows the strap portion to be tightened to the head portion at a desired position, a loop having a desired size composed of a distal end side part of the strap portion and the head portion can be formed when tightened by the locking feature, and a plurality of features for keeping connected to thread are along the longitudinal direction on at least the distal end side part of the strap portion that forms the loop.
[2] The medical instrument according to [1], wherein the locking feature is composed of a combination of at least one ratchet pawl provided on the head portion and a plurality of ratchet teeth provided along the longitudinal direction on the strap portion; a combination of at least one depression provided in the head portion and a plurality of protrusions provided along the longitudinal direction of the strap portion; or a combination of at least one protrusion provided on the head portion and a plurality of depressions provided on the strap portion along the longitudinal direction.
[3] The medical instrument according to [1] or [2], wherein the features for keeping connected to thread are arranged so that the thread can be connected almost evenly around the entire circumference of the loop.
[4] The medical instrument according to any one of [1] to [3], wherein the feature for keeping connected to thread comprises a hook, an arch, a loop, a through hole, a plexus or a reticulation.
[5] The medical instrument according to any one of [1] to [4], wherein the feature for keeping connected to thread is configured so that a suture thread does not touch living tissue when the suture thread is passed through the feature for keeping connected to thread.
[6] The medical instrument according to [5], wherein the feature for keeping connected to thread comprises a hook.
[7] The medical instrument according to [6], wherein the hook has a gate.
[8] The medical instrument according to [5], wherein the feature for keeping connected to thread comprises a through hole that penetrates from the outer surface to the side surface of the strap portion and / or the head portion.
[9] The medical instrument according to [8], wherein the through-hole is a straight hole.
[10] The medical instrument according to [8], wherein the through hole is a curved hole.
[11] The medical instrument according to [5], wherein the feature for keeping connected to thread comprises a through hole C-shaped with a slit.
[12] The medical instrument according to [11], wherein a gate is provided in the slit.
[13] The medical instrument according to any one of [1] to [12], further comprising a berm along the longitudinal direction of the strap portion between the feature for keeping connected to thread and the edge of the strap portion.
[14] The medical instrument according to any one of [1] to [13], wherein the strap portion, the head portion and the tail portion are composed of a biodegradable and bioabsorbable polymer.
[15] The medical instrument according to any one of [1] to [14], configured for ligating and/or suturing a living tissue.
[16] The medical instrument according to [15], wherein the living tissue is an organum or an organ.

### ADVANTAGEOUS EFFECTS OF THE INVENTION

The medical instrument of the present invention can be used to prevent the occurrence of various complications in partial resection or the other operations of living tissue (an organ, organum) such as liver, pancreas and blood vessels, and is less invasive and suitable for ligation, suture, fixation, etc.

When the medical instrument of the present invention is attached to a living tissue, the features for keeping connected to thread are arranged evenly over the entire circumference of the living tissue. In addition, the features for keeping connected to thread facilitates connecting a suture thread to the strap portion and head portion of the medical instrument attached to a living tissue without passing a suture needle or the suture thread through the living tissue. By using the medical instrument of the present invention, ligation, suture or fixation of a living tissue can be easily performed in a short time without almost damaging the living tissue.

### BRIEF DESCRIPTION OF THE DRAWINGS

FIG. 1 is a view showing an example of the medical instrument of the present invention.
FIG. 2 is a view showing an A-A cross section of the strap portion of the medical instrument shown in FIG. 1.
FIG. 3 is a view showing a B-B cross section of the head portion of the medical instrument shown in FIG. 1.
FIG. 4 is a view showing a B-B cross section in a state in which the strap portion is tightly bound to the head portion.
FIG. 5 is a view showing another example of the medical instrument of the present invention.
FIG. 6 is a view showing another example of the medical instrument of the present invention.
FIG. 7 is a view showing another example of the medical instrument of the present invention.
FIG. 8 is an enlarged view of the part of the feature for keeping connected to thread in FIG. 7.
FIG. 9 is a view showing another example of the medical instrument of the present invention.
FIG. 10 is an enlarged view of the part of the feature for keeping connected to thread in FIG. 9.
FIG. 11 is a view showing the pancreas (right) from which the pancreatic head has been resected and the jejunum (left). The medical instrument 17 of the present invention is shown in a state entering the dorsal side of the pancreas and preparing to attach the stump.
FIG. 12 is a view showing a state in which the medical instrument of the present invention is attached to the stump of the pancreas.
FIG. 13 is a view showing a state in which the pancreatic stump and the jejunum are sutured with the suture thread.
FIG. 14 is an enlarged view of the sutured portion in FIG. 13.
FIG. 15 is a view showing another example of the medical instrument of the present invention.
FIG. 16 is a partially enlarged view showing another example of the feature for keeping connected to thread.
FIG. 17 is a view showing another example of the medical instrument of the present invention.
FIG. 18 is a view showing an A-A cross section of the strap portion of the medical instrument shown in FIG. 17.
FIG. 19 is a view showing an example of a through hole with a slit.

### EMBODIMENTS FOR CARRYING OUT THE INVENTION

The medical instrument of the present invention comprises a strap portion 1, a head portion 2 and a tail portion 3.

The strap portion 1 is a line-like or belt-like portion flexible and has a distal end and a proximal end. The flexibility of the strap portion may be due to the flexibility of the material itself, may be based on a mechanical structure that allows bending. The head portion 2 is a portion that connects to the distal end of the strap portion 1 and extends in the distal direction from the distal end of the strap portion. The tail portion 3 is a portion that connects to the proximal end of the strap portion and extends in the proximal direction from the proximal end of the strap portion. The strap portion 1, the head portion 2 and/or the tail portion 3 are preferably composed of a medical material, more preferably composed of a biocompatible polymer, and even more preferably composed of a biodegradable and bioabsorbable polymer. The tail portion 3 is a leading part that leads the strap portion, for example, when the strap portion will be tightened to the head portion 2. The tail portion 3 may be provided with ribs or the like along the longitudinal direction to make it difficult to buckle.

The strap portion, the head portion and the tail portion may be formed in bulk, may be made of fibers such as nets, woven fabrics, non-woven fabrics, and the like. In order to make it difficult to bend, it is preferable to use a bulk formation.

In addition, for example, the medical instrument of the present invention can be obtained by molding a polymer being a medical material by a known resin molding method to form the shape of each portion. Examples of the polymer can include lactic acid polymer, lactic acid-glycolic acid polymer, trimethylene carbonate-based polymer, dioxanone-based polymer, polyethylene glycol-based polymer, and lactone-based polymer.

Also, the strap portion, the head portion and the tail portion are not particularly limited in terms of their length, thickness, elastic modulus, or the like. The length, thickness, elastic modulus, and the like can be appropriately set according to, for example, the shape and size of the living tissue to be treated.

The head portion and the strap portion have locking feature. The locking feature can tighten the strap portion to the head portion at a desired position.

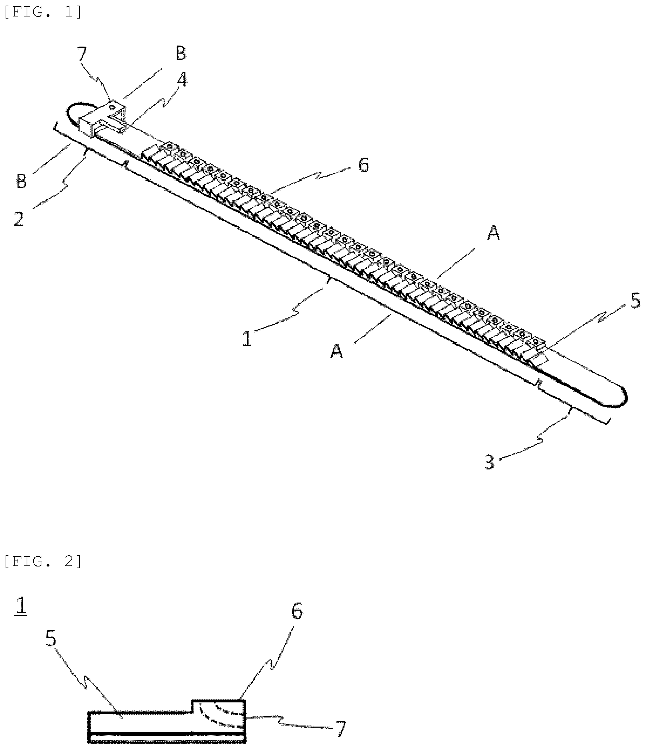

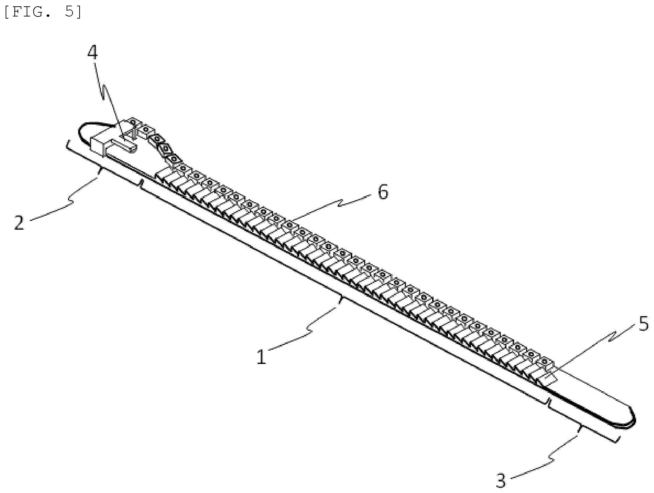

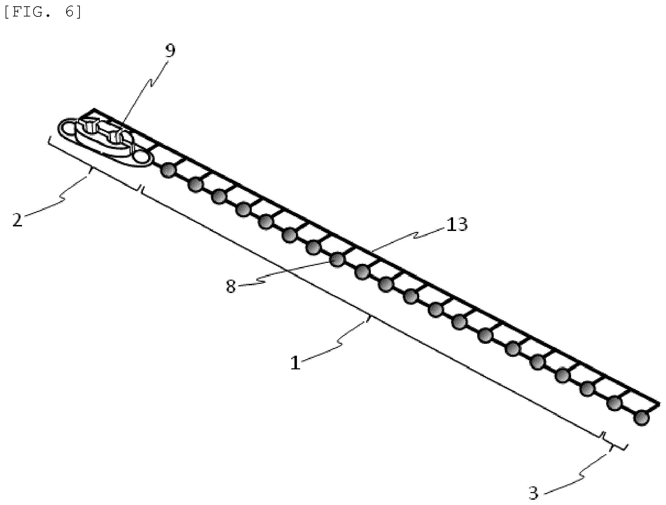

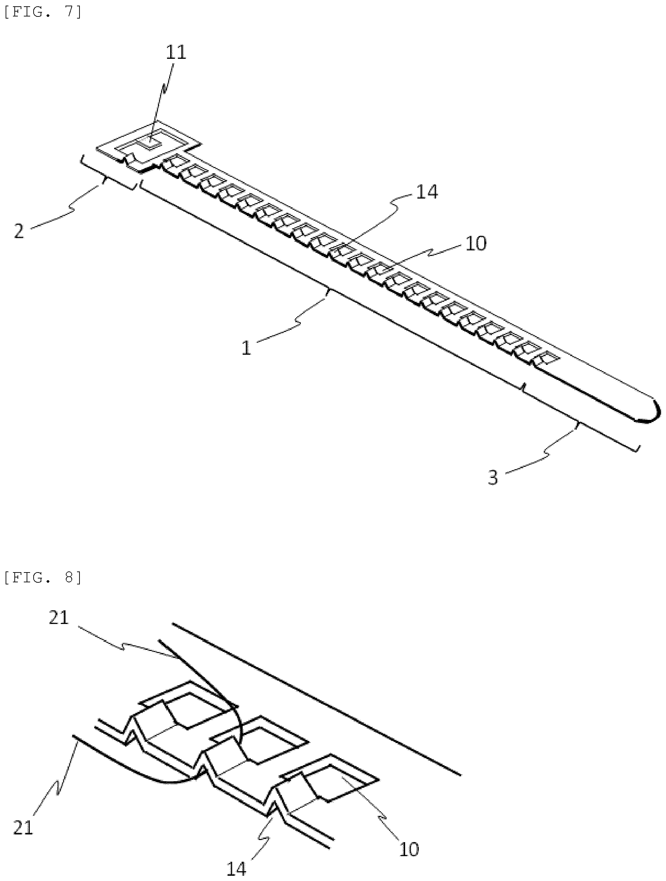

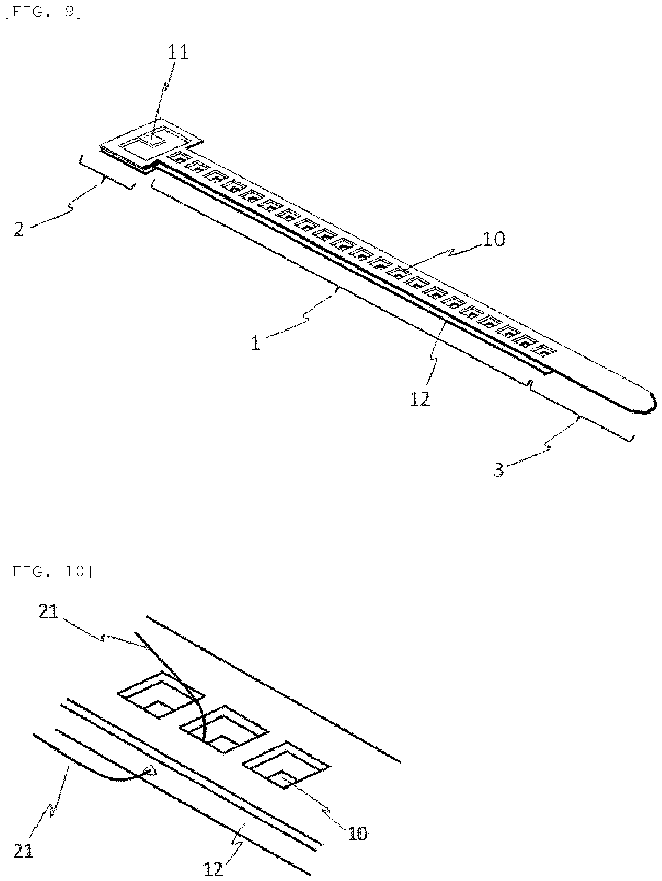

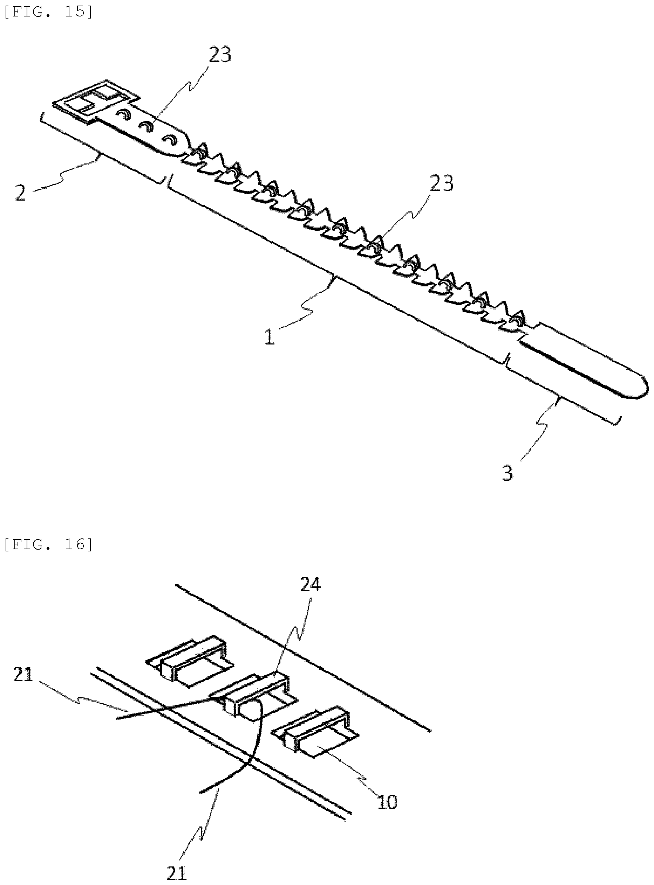

The locking feature consists of a combination of a part on the strap portion and a part on the head portion configured to be tightened to the part on the strap portion. As the locking feature, mentioned can be, for example, a combination of at least one ratchet pawl 4 provided on the head portion and a plurality of ratchet teeth 5 provided along the longitudinal direction of the strap portion (see FIG. 1 or FIG. 5), a combination of at least one depression provided in the head portion and a plurality of protrusions provided along the longitudinal direction of the strap portion (see FIG. 6 or FIG. 15); or a combination of at least one protrusion provided on the head portion and a plurality of depressions provided along the longitudinal direction of the strap portion (see FIG. 7 and FIG. 9).

A pin, a hook, etc. can be mentioned as a protrusion in the locking feature. As the depression in the locking feature, mentioned can be a hole, a loop, a constriction, a notch, or the like. The plurality of protrusions or depressions provided on the strap portion may be a string of spherical or annular objects (see FIG. 6), saw-toothed objects on both sides (see FIG. 15), or through holes provided along the longitudinal direction (see FIG. 7 and FIG. 9).

The protrusion in the locking feature preferably has a barb to prevent it from slipping out of the depression into which it is inserted. The barbed protrusion may be arrowhead-shaped, hook-shaped, T-shaped, inverted L-shaped, or the like. The plurality of holes provided in the head portion or the strap portion may be reticulations in cloth or net as long as the protrusion can be inserted through them. Also, as a combination of hook and loop, for example, hook-and-loop fastener may be used.

A ratchet, which is an example of a locking feature, is one of the mechanisms used to limit the direction of movement to one direction. Tightening can be achieved by engaging the ratchet tooth 5 with the ratchet pawl 4. The ratchet pawl is classified into a movable type and a fixed type. When the movable ratchet pawl is engaged with the ratchet tooth, the engagement can be easily released. When the fixed ratchet pawl engages the ratchet tooth, it is difficult to release the engagement. In the drawings, one movable ratchet pawl is provided. When both of a fixed ratchet pawl and a movable ratchet pawl are installed, the strap portion is firstly locked with the movable ratchet pawl, the engagement can be released to loosen the tightening when the tightening force on the living tissue is too large, then, the fixed ratchet pawl can be engaged so that the position of the strap portion does not change when the tightening force has been determined.

When tightened by the locking feature, the head portion 2 and a distal end side part of the strap portion can form a loop having desired size, wherein the distal end side part of the strap portion is specifically a part from the locking position of the strap portion to the distal end of the strap portion that is a position just before the head part 2.

By changing the locking position, the distal end side part of the strap portion forming a part of the loop can be changed in length; as the result, the loop size can be changed. This allows the loop to have a desired size and a desired tightening force depending on the size of the object to be ligated.

A surface of the strap portion or the head portion to touch the living tissue may be a rough surface, a surface with convex or concave striae or grids, or a surface having a plurality of convex or concave points, in order to increase frictional force with the living tissue and prevent slipping during ligation. By providing convex striae parallel to the longitudinal direction near the edge of the surface that touches the living tissue, the contact between the living tissue to be ligated and the medical instrument of the present invention can be enhanced.

Furthermore, in order to make the living tissue less likely to be damaged when it is tightened, a layer of cushioning material 12 may be attached to the tissue-contacting surface of the strap portion or the head portion. Examples of cushioning materials can include soft elastic bodies such as rubber, foamed elastic bodies such as sponge, non-woven fabrics such as felt and woven fabrics. The cushioning material is preferably composed of a medical material, more preferably composed of a biocompatible polymer, and even more preferably composed of a biodegradable and bioabsorbable polymer.

The medical instrument of the present invention has a plurality of features for keeping connected to thread (thread tethering features) along the longitudinal direction on at least a loop-forming part of the distal end side of the strap portion, and, if necessary, a loop-forming part of the head portion. The thread tethering features are arranged along the circumference of the formed loop when engaged by the locking feature. The thread tethering features are preferably arranged along the circumference of the formed loop so that the thread can be tethered substantially evenly around the entire circumference of the loop. The thread tethering feature facilitates tethering a suture needle or suture thread to the strap portion and the head portion of the medical instrument attached to a living tissue without penetrating the living tissue.

As the thread tethering feature, mentioned can be, for example, a hook, a bitt or bollard, a through hole, a loop, an arche, a plexus or reticulation, and the like.

A hook, bitt or bollard as the thread tethering feature may be provided with a barb or a gate such as a carabiner so that the passed thread does not come off.

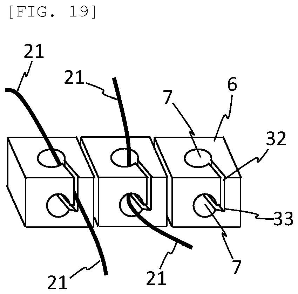

The through-hole as the thread tethering feature may penetrate from the outer surface to the inner surface of the strap portion and the head portion, or may penetrate from the outer surface to the side surface of the strap portion and the head portion. The through hole 7 may have a slit 32 in the peripheral wall of the through hole. As shown in FIG. 19, in the through hole 7 having the slit 32, the suture thread 21 can be reeved through the slit 32 from the side of the through hole 7 without entering through the entrance of the through hole 7. The slit may be provided with a barb, a bent section 33, a gate like a carabiner, or the like. The barb, bent section or gate can prevent the reeved thread from exiting the through-hole via the slit.

In the medical instrument shown in FIG. 1, as shown in FIG. 2, cuboid blocks are provided along the right side from the proximal end to the distal end of the strap section, and the cuboid block has a through-hole 7 that penetrates from the top surface to the right side surface of the cuboid block. Since it is divided into the cuboid blocks, even if the thickness of the cuboid block increases, the flexibility of the strap portion can be maintained at a high level. The part where the through hole is provided is not limited to a rectangular parallelepiped shape, for example, a quadratic prism, a triangular prism, a circular cylinder, or a hemisphere may be employed.

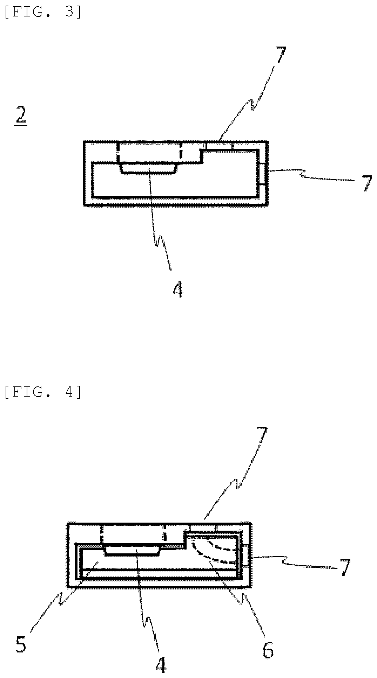

Moreover, as shown in FIG. 3, through holes 7 are provided on the right upper surface and the right side surface of belt loop having the ratchet pawl 4 on the left side. When the strap portion is passed through the belt loop with the ratchet pawl 4 on the left side, as shown in FIG. 4, spacing between engagement positions of the ratchet pawl and the ratchet teeth is preferably set so that the through hole 7 provided in the belt loop of the head portion overlaps with the through hole provided in the cuboid block 6 of the strap portion. Note that the cuboid block may be provided on the left side instead of on the right side, or may be provided on both of the left side and the right side.

The medical instrument shown in FIG. 5 has the same structure as the medical instrument shown in FIG. 1 except that the cuboid blocks 6 are provided not only in the strap portion but also in the head portion. The medical instrument shown in FIG. 5 can tether the suture substantially evenly around the entire circumference of the medical instrument. Therefore, the medical instrument of the present invention is excellent in that the living tissue can be sutured and fixed substantially evenly over the entire circumference of the ligated organ.

In the medical instrument shown in FIG. 6, the strap portion is formed by connecting spherical bodies in a string. And, the thread tethering feature is configured by connecting the spherical bodies 8 and the linear member 13 so as to form through spaces that lie in a ladder shape. A needle or thread can be passed through the through space. In addition, the thread tethering feature is provided on the left side from the distal end to the proximal end of the strap portion in FIG. 6. It may be provided on the right side instead of the left side or may be provided on both of the left and right sides. By fitting the spherical body 8 into the depression 9 provided in the head portion 2, the strap portion can be tightened to the head portion at a desired position. In the thread tethering feature formed by the linear member 13 connected to the spherical bodies 8, even when the strap portion is wrapped and tightened around the living tissue, a space through which a needle or thread can be passed can be created between the living tissue and the medical instrument by raising the linear member 13. Therefore, suturing can be performed without damaging the living tissue.

In the medical instrument shown in FIG. 7, the strap portion comprises a band-shaped member having through holes 10 lied in a ladder shape, the strap portion can be tied to the head portion by inserting protrusion 11 provided on the head portion into one of the ladder-shaped through holes 10 on the strap portion. The ladder-shaped through-hole has a notch 14 provided on the right side of the strap portion from the distal end to the proximal end. When the medical instrument shown in FIG. 7 is wrapped around the living tissue, the notch 14 constitutes a thread tethering feature. In the embodiment of the invention shown in FIG. 7, the ladder-shaped through-hole constituting a part of the locking feature also functions as the thread tethering feature. In the thread tethering feature formed by the notch 14, as shown in FIG. 8, the notch creates a space between the living tissue and the medical instrument through which the needle and thread can be passed, and the medical instrument of the present invention can be connected to a thread without penetrating the living tissue with a needle or the thread. The shape of the notch is not limited to the triangular shape shown in the figure, and may be rectangular shape, trapezoidal shape, semicircular shape, sinusoidal waveform, rectangular waveform or the like. In addition, though the thread tethering feature is provided on the right side from the distal end to the proximal end of the strap portion in FIG. 7, it may be provided on the left side instead of the right side, or may be provided on both of the left and right sides.

The medical instrument shown in FIG. 9 is provided with a layer 12 of cushioning material on the inner surface (bottom surface in the figure) of the strap portion. As shown in FIG. 10, there are many voids in the layer of cushioning material such as felt, by passing a suture needle and a suture thread through the void, the suture thread can be connected to the medical instrument of the present invention without the suture needle and the suture thread penetrating a parenchyma of the living tissue.

In the medical instrument shown in FIG. 15, loops 23 as a thread tethering feature are provided along the longitudinal direction on the head portion 2 and the outer surface (upper surface in the figure) of the strap portion 1. By passing a suture needle and suture thread through the loop 23, the suture thread can be connected to the medical instrument of the present invention without the suture needle and the suture thread penetrating a parenchyma of the living tissue.

FIG. 16 shows a modified example in the ladder-shaped through-hole of the medical instrument shown in FIG. 9. In this medical instrument, lintel 24 is provided above the ladder-shaped through holes. By passing a suture thread 21 under the lintel 24, the suture thread can be connected to the medical instrument of the present invention without the suture needle and the suture thread penetrating a parenchyma of the living tissue. The orientation of the lintel is not limited to that shown in FIG. 16, the lintel may be oriented parallel to the longitudinal direction. It can be understood that the lintels 24 shown in FIG. 16 can be replaced with arches to achieve the same effect.

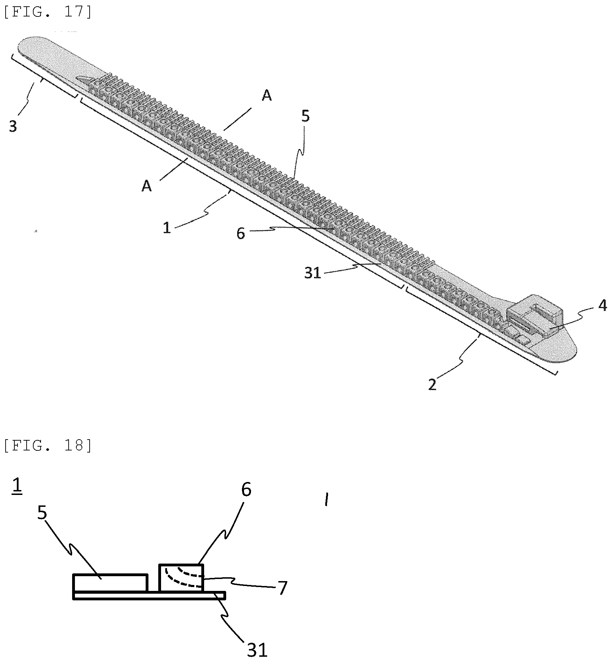

FIG. 17 is a view showing another example of the medical instrument of the present invention. FIG. 18 is a view showing a A-A cross section of the strap portion of the medical instrument shown in FIG. 17. The medical instrument shown in FIG. 17 has the same structure as the medical instrument shown in FIG. 1 except that a berm (scarcement, band for anti-occlusion of through-holes) 31 is provided along the longitudinal direction between the cuboid block 6 and the edge of the strap portion, the placement of the cuboid block and the shape of the locking feature have been changed.

The berm 31 can secure a space between the side surface of the cuboid block 6 and the edge of the strap portion, it is possible to prevent the through hole 7 on the side surface from being occluded by embossment of fresh when the medical instrument is wrapped around the living tissue. This berm can also be employed in the other thread tethering features.

The medical instrument of the present invention, for example, in pancreatic resection, can be preferably used in medical acts like pancreatoenterostomy such as pancreatojejunostomy or pancreatogastrostomy.

The pancreatoenterostomy itself is known as a conventional technique. For example, the methods for the pancreatoenterostomy are described in Owada et al., "DEVISAL OF PANCREATICOJEJUNOSTOMY IN PANCREATICODUODENECTOMY" Kita Kanto Medicine 43(1) 63-68, 1993; Shinchi et al., "SURGICAL TECHNIQUE FOR PANCREATOENTEROSTOMY IN PANCREATICODUODENECTOMY - SAFE AND RELIABLE PANCREATICOGASTROSTOMY -" Med. J Kagoshima Univ., Vol. 64, No. 1-2, 1-7, September, 2012; Jinnai "GASTROINTESTINAL ANASTOMOSIS TECHNIQUE" Journal of the Japanese Society of Clinical Surgery No. 1 the 40th General Assembly Educational Lecture P17-23 or the like.

In the present invention, pancreatojejunostomy in pancreatic head resection using the medical instrument of the present invention, for example, can be done as follows.

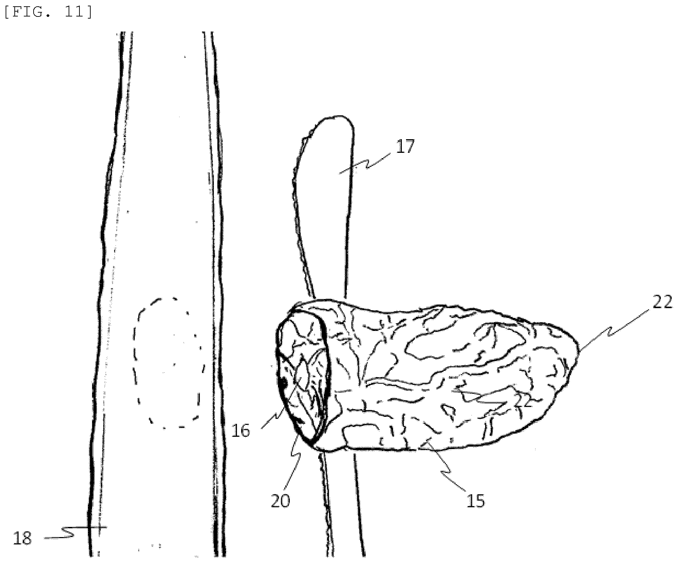

First, the pancreatic head is resected. The cut surface can then be subjected to sutured hemostasis or electro-hemostasis as appropriate, but the stump 20 has not yet been closed. An incision is made at the portion of the jejunum 18 that is to be anastomosed. As a stent or the like, if necessary, a pancreatic duct tube is passed through the incised portion of the jejunum to allow communication between the inside and the outside of the jejunum (FIG. 11).

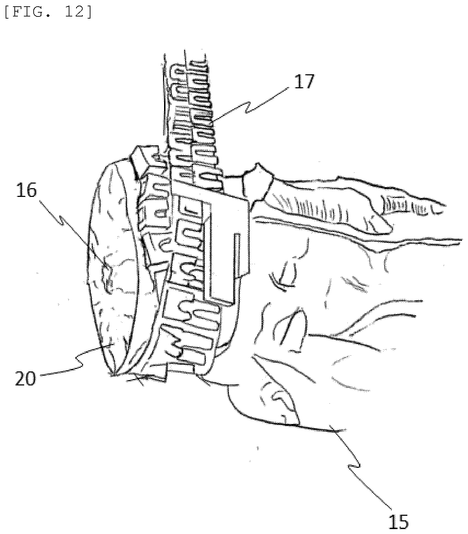

The medical instrument 17 of the present invention is attached to the stump of the pancreas, and the pancreatic stump 20 is wrapped at a strength that narrows the micro pancreatic duct but does not narrow the main pancreatic duct 16 (FIG. 12). Compressing the pancreatic parenchyma of the stump can suppress the leakage of pancreatic juice from openings of the pancreatic duct and the pancreatic parenchyma exposed at the stump.

A suture needle is used to pass the suture thread 21 through both the posterior (dorsal side) the jejunum and the thread tethering feature (the through hole of the cuboid block 6 in the figure) of the medical instrument of the present invention on the posterior (dorsal side) of the stump of the pancreas. The suture thread is then pulled to bring the posterior (dorsal side) of the jejunum into close contact with the dorsal side of the stump of the pancreas.

The main pancreatic duct 16 and the incised portion of the jejunum are anastomosed after the pancreatic tube that has passed through the incised portion of the jejunum as necessary is inserted into the main pancreatic duct 16.

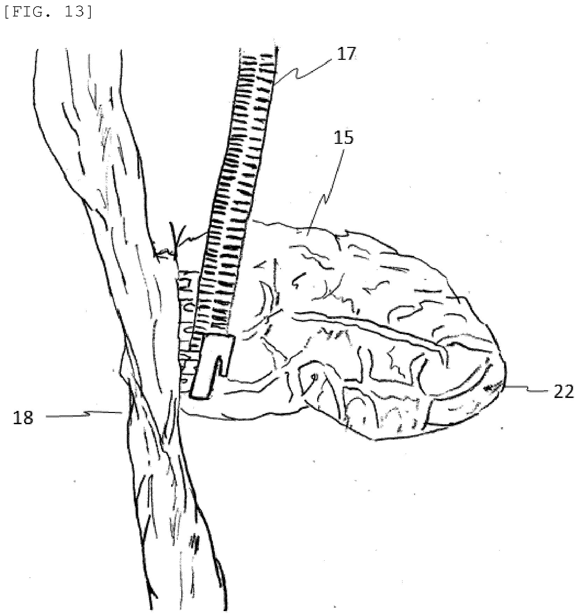

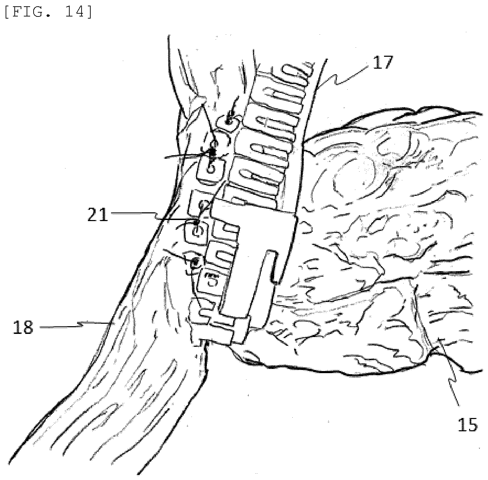

Then, the suture needle is used to pass the suture thread 21 through both the topside (cephalic side) of the jejunum and the thread tethering feature (the through hole of the cuboid block 6 in the figure) of the medical instrument of the present invention on the topside (cephalic side) of the stump of the pancreas. The suture thread is then pulled to bring the wall of the jejunum into close contact with the topside (cephalic side) of the stump of the pancreas (FIG. 13, FIG. 14). The same operations are performed on the downside (caudal side) and anterior (ventral side) of the stump of the pancreas. Thus, by using the medical instrument of the present invention, the suture needle and suture thread are passed through the thread tethering feature to connect the suture thread at the thread tethering feature, the pancreatic stump can be circumferentially covered with the jejunum without penetrating the pancreatic parenchyma with the suture needle or suture thread.

The medical instrument of the present invention is, in the partial excision of a living tissue, suitably used for medical acts such as ligation of the targeted living tissue, fixation or suturing of other living tissues or other organs, and the like. Examples of the living tissue to be ligated using the medical instrument can include an organum or an organ. Examples of the organum can include an organum having a tubular structure such as blood vessel, lymphatic vessel, thoracic duct, bile duct, fallopian tube, vagina, ureter, urethra, vas deferens, trachea, and bronchi. Examples of the organ can include pancreas, liver, gallbladder, spleen, lien, kidney, bladder, uterus, ovary, testicle, lung, heart, thyroid, esophagus, stomach, duodenum, small intestine, large intestine, and lymph node. Of these, spleen, kidney, liver or pancreas are preferred, pancreas is more preferred, body or tail of the pancreas is particularly preferred. As a medical act such as suturing and fixing, mentioned can be, for example, pancreatoenterostomy such as pancreatojejunostomy, pancreaticogastrostomy, and the like. Of these, pancreatojejunostomy or pancreaticogastrostomy is preferred.

For example, when suturing pancreas and jejunum in pancreatojejunostomy, in the prior art, a suture needle and suture thread have to pierce the pancreas directly to suture it to the jejunum. At this time, the suture needle and suture thread inserted into the pancreas damage the pancreatic tissue or pancreatic duct, causing pancreatic fistula. In addition, when the suture thread inserted into the pancreas and the jejunum is ligated, the suture thread tears and damages the pancreatic tissue and pancreatic duct, which also causes pancreatic fistula. Insufficient ligation strength at the suture of the pancreas and jejunum, insufficient contact between the pancreatic stump and the jejunum, or insufficient sealing of the pancreatic stump with the jejunum can also lead to pancreatic fistula. When pancreatic fistula develops, there is a risk of complications such as intra-abdominal abscess or pseudoaneurysm, leading to death, so prevention of pancreatic fistula is a very important issue. In addition, if the ligation to the pancreas is too strong, the ligated tissue may become necrotic, so there is also a demand for minimal invasiveness. Furthermore, contents may leak out from a hole in the living tissue opened by the suture needle, causing damage to the living tissue.

The medical instrument of the present invention can solve such problems. Specifically, the medical instrument of the present invention can also be used to prevent the occurrence of various complications in partial excision or the like of living tissue. The medical instrument of the present invention has the thread tethering feature configured to pass a suture needle and a suture thread, it is possible to fix an organum or an organ to the pancreas without directly inserting the suture needle or suture thread into the pancreatic tissue. More specifically, the medical instrument of the present invention is ligated and fixed to the pancreas with the suture needle and suture thread inserted into the holes of the thread tethering feature provided in the instrument, and the suture needle and suture thread are inserted to the organum or organ to be sutured or fixed to the pancreas to ligate the organum or organ and the pancreas. As a result, damage to the pancreatic tissue and pancreatic duct due to insertion or ligation of suture needles and suture thread can be prevented, and the risk of developing pancreatic fistula can be reduced.

The medical instrument of the present invention can contribute to less invasiveness because the suture needle and suture thread used for suturing can be connected via the thread tethering feature without touching the ligated living tissue. Examples of such thread tethering feature can include a hook such as a hook with a gate, and a through hole penetrating from the outer surface to the side surface of the strap portion and/or the head portion. A plurality of thread tethering features that can achieve such minimal invasiveness are arranged so that the thread can be tied evenly around the circumference of the loop formed, resulting in circumferential fixation of the organum or organ as well as prevention of uneven tension of the suture applied to the ligated living tissue, and these points can also contribute to low invasiveness.

The medical instrument of the present invention is not limited to the embodiments shown in the drawings. The technical scope of the present invention also includes change of the shape, size, color or material of each of the above members, or addition of well-known or commonly used parts other than the above-mentioned members.

### CODE EXPLANATION

1: Strap portion
2: Head portion
3: Tail portion
4: Ratchet pawl
5: Ratchet teeth
6: Thread tethering feature (cuboid block with through holes)
7: Through hole
8: Spherical body
9: Depression corresponding to the spherical body
10: Ladder-shaped through hole
11: Protrusion corresponding to the ladder-shaped through-hole
12: Layer of cushioning material (felt)
13: Thread tethering feature (linear member that forms the ladder-shaped through-hole)
14: Thread tethering feature (notch)
15: Body of pancreas
16: Opening of the main pancreatic duct
17: Medical instrument of the present invention
18: Jejunum
20: Stump after excision of head of the pancreas
21: Suture thread
22: Tail of pancreas
23: Thread tethering feature (loop)
24: Thread tethering feature (lintel)
31: Berm
32: Slit
33: Bent section

## Claims

1. A medical instrument, comprising a flexible strap portion having a proximal end and a distal end; a head portion distally extending from and connected to the distal end of the strap portion; and a tail portion proximally extending from and connected to the proximal end of the strap portion; wherein the head portion and the strap portion have a locking feature that allows the strap portion to be tightened to the head portion at the desired position, a loop having a desired size composed of a distal end side part of the strap portion and the head portion can be formed when tightened by the locking feature, and a plurality of features for keeping connected to thread are along the longitudinal direction on at least the distal end side part of the strap portion that forms the loop.

2. The medical instrument according to claim 1, wherein the locking feature is composed of a combination of at least one ratchet pawl provided on the head portion and a plurality of ratchet teeth provided along the longitudinal direction on the strap portion; a combination of at least one depression provided in the head portion and a plurality of protrusions provided along the longitudinal direction of the strap portion; or a combination of at least one protrusion provided on the head portion and a plurality of depressions provided on the strap portion along the longitudinal direction.

3. The medical instrument according to claim 1 or 2, wherein the feature for keeping connected to thread are arranged so that the thread can be connected almost evenly around the entire circumference of the loop.

4. The medical instrument according to any one of claims 1 to 3, wherein the feature for keeping connected to thread comprises a hook, an arch, a loop, a through hole, a plexus or a reticulation.

5. The medical instrument according to any one of claims 1 to 4, wherein the feature for keeping connected to thread is configured so that a suture thread does not touch living tissue when the suture thread is passed through the feature for keeping connected to thread.

6. The medical instrument according to claim 5, wherein the feature for keeping connected to thread comprises a hook.

7. The medical instrument according to claim 6, wherein the hook has a gate.

8. The medical instrument according to claim 5, wherein the feature for keeping connected to thread comprises a through hole that penetrates from the outer surface to the side surface of the strap portion and / or the head portion.

9. The medical instrument according to claim 8, wherein the through-hole is a straight hole.

10. The medical instrument according to claim 8, wherein the through hole is a curved hole.

11. The medical instrument according to claim 5, wherein the feature for keeping connected to thread comprises a through hole C-shaped with a slit.

12. The medical instrument according to claim 11, wherein a gate is provided in the slit.

13. The medical instrument according to any one of claims 1-12, further comprising a berm along the longitudinal direction of the strap portion between the feature for keeping connected to thread and the edge of the strap portion.

14. The medical instrument according to any one of claims 1 to 13, wherein the strap portion, the head portion and the tail portion are composed of a biodegradable and bioabsorbable polymer.

15. The medical instrument according to any one of claims 1 to 14, configured for ligating and/or suturing a living tissue.

16. The medical instrument according to claim 15,
wherein the living tissue is an organum or an organ.
